# EUROPEAN PATENT SPECIFICATION

(11) **EP 4 065 083 B1**
(45) Date of publication and mention of the grant of the patent: **09.08.2023**
(21) Application number: 20816124.0
(22) Date of filing: 23.11.2020
(51) Int. Cl.: A61K 9/20, A61K 9/00, A61K 9/70, A61K 47/36, A61K 47/42, B29C 64/106, B33Y 10/00, B33Y 70/00, B33Y 80/00

(54) **COMPOSITION FOR THE 3D PRINTING OF SEMISOLID DRUGS**
ZUSAMMENSETZUNG ZUM 3D-DRUCKEN VON HALBFESTEN ARZNEIMITTELN
COMPOSITION POUR L'IMPRESSION 3D DE MÉDICAMENTS SEMI-SOLIDES

(30) Priority: 26.11.2019 ES 201931041
(43) Date of publication of application: 05.10.2022
(73) Proprietor: Fundacion Idonial, 33203 Gijón (Asturias) (ES)
(72) Inventor: HERRADA MANCHÓN, Helena, 33203 Gijón (Asturias) (ES); FERNÁNDEZ HERNÁNDEZ, Manuel, Alejandro, 33203 Gijón (Asturias) (ES)
(74) Representative: Clarke Modet & Co.
(86) International application number: PCT/EP2020/083042
(87) International publication number: WO 2021/105051

(56) References cited:
- EP-A1- 3 403 643
- US-A1- 2018 263 904
- GOYANES ALVARO ET AL: "Automated therapy preparation of isoleucine formulations using 3D printing for the treatment of MSUD: First single-centre, prospective, crossover study in patients", INTERNATIONAL JOURNAL OF PHARMACEUTICS, ELSEVIER, NL, vol. 567, 4 July 2019 (2019-07-04), XP085741507, ISSN: 0378-5173, DOI: 10.1016/J.IJPHARM.2019.118497 [retrieved on 2019-07-04]
- SCOUTARIS NICOLAOS ET AL: "3D Printed "Starmix" Drug Loaded Dosage Forms for Paediatric Applications", PHARMACEUTICAL RESEARCH, SPRINGER NEW YORK LLC, US, vol. 35, no. 2, 16 January 2018 (2018-01-16), pages 1-11, XP036435304, ISSN: 0724-8741, DOI: 10.1007/S11095-017-2284-2 [retrieved on 2018-01-16]

## Description

### TECHNICAL FIELD OF THE INVENTION

The present invention falls within the technical field of pharmaceutical technology. Specifically, the invention relates to semisolid compositions and their use as ink in 3D printing devices for the personalized dosing of drugs and/or active pharmaceutical ingredients (APIs).

### BACKGROUND OF THE INVENTION

3D printing, also known as additive manufacturing, has revolutionized all technological fields. This technology refers to the creation of a three-dimensional object from a digital design by means of successively superimposing layers of material. Its applications are increasingly expanding and range from the generation of simple, daily objects to applications in high-impact sectors such as aeronautics, health or the automotive industry.

One of the latest sectors to be incorporated in 3D printing is the chemical-pharmaceutical sector. In this sense, one of the most important potential applications of this technology is the printing of medicinal products, with a clear initial objective focused on finding ways for the personalized dosing of the active ingredients present therein. The dose of a specific active ingredient can thereby be adjusted based on the needs of each patient. Furthermore, personalized dosing could be the starting point for another vitally important milestone: multi-pills. Multi-pills would allow doses of different active ingredients to be delivered in a single tablet, thereby improving therapeutic compliance and the quality of life of the patient by reducing the number of pills to be taken each day.

The standard ISO 17296-2:2015 describes commercially available 3D printing technologies which, in practice, have proven to be useful and viable on the market for several years. Although seven main categories of technologies, within pharmaceutical products, have been classified, only the following have been researched:
- Binder jetting: additive manufacturing process in which a liquid binding agent is selectively deposited for bonding powder materials.
- Material extrusion: additive manufacturing process in which the material is selectively dispensed through a nozzle or a port.
   - Fused deposition modelling (FDM), in which printing is done from a solid filament of heat-sensitive material.
   - Semisolid extrusion (SSE), in which printing is done by means of semisolid materials, such as gels or pastes.
- Powder bed fusion: additive manufacturing process in which thermal energy selectively melts certain regions of a powder bed. An example would be selective laser sintering (SLS), which uses laser energy to melt the powder.
- Photopolymerization in tank or vat: additive manufacturing process in which a liquid photopolymer is selectively cured in a vat by means of light-activated polymerization. An example of this type of printing is stereolithography (SLA), where the ultraviolet (UV) laser is focused on the top part of the photopolymerizable liquid.

Although within additive manufacturing, material extrusion technologies have been the object of most of the research to date, the only medicinal product on the market generated by additive manufacturing is made by means of binder jetting. This drug, called Spritam^{®} (Aprecia Pharmaceuticals, East Windsor, NJ, USA), is a solid, rapidly dispersing pill containing an active ingredient for the treatment of epilepsy (levetiracetam). Patent document WO2014143935 describes the formulation of this medicinal product, which has 3D-printed porous matrix comprising drug particles of a specific size, at least one disintegrant, at least one surfactant and at least one binder. Spritam^{®} is mass produced like a conventional drug, i.e., hundreds of pills are produced with the same dose and the same active ingredient, which means that it is not possible to manufacture tablets with personalized doses or with mixtures of multiple active ingredients for any patient. Although printing with different doses of the active ingredient will be selected so as to customize it to patients with different needs, modifications would be required both in the production line and in the formulation of the rest of the components of the medicinal product, so versatility of the technique would be very limited.

A pharmaceutical form (or galenic form) is a substance or an association of substances, the purpose of which is to make it easier to administer drugs to the body. It is the physical arrangement to which the active ingredients and excipients present in a medicinal product are adapted. Pharmaceutical forms, according to their physical state, can be liquid forms (syrups, elixirs, lotions, tinctures, etc.), solid forms (granulates, powders, wafers, cachets, pessaries, capsules, suppositories, etc.), semisolid forms (salves, gels, creams, pastes, etc.) or gaseous forms (inhalers, sprayers, aerosols, etc.).

Patent document EP1503741 describes a dosage form made up of an active ingredient-containing core and an outer layer which blocks its release but allows its diffusion once activated, allowing the controlled release of the drug. It is a tablet-type, solid pharmaceutical form, the novelty of which resides in its API release profile. Solid pharmaceutical forms are defined as pharmaceutical preparations comprising a dose of one or several active ingredients obtained by the uniform compression of particles and moulding, which must be swallowed whole. Having to take them whole is a drawback for patients who have problems with swallowing or who are not cooperative, such as children and the elderly, for example. This can be a serious problem, as it could seriously compromise compliance, on the part of the patient, with the treatment prescribed by a doctor, thereby compromising their improvement. This lack of desire to swallow solid forms is also common in healthy adults who, due to a lack of trust, do not talk to their doctor and end up failing in their treatment without the doctor being able to consider other alternatives.

Systems which describe compositions and methods of manufacture based on photopolymerization techniques are also known. For example, patent document CN106008850 describes a hydrogel that can be loaded with different active ingredients and manufactured by stereolithography (SLA) printing. Stereolithography requires the use of a liquid resin which polymerizes by means of a laser beam. That means, on one hand, that the resin cannot be toxic or represent any problem if a patient ingests it, and complete elimination thereof must be ensured, which is a difficult task because there is still no regulation that addresses this process or the acceptable percentages of "residual" resin in the medicinal product. On the other hand, the effect of the laser on the active ingredient entails a risk of modifying its chemical structure and/or decomposing the active ingredient which needs to be thoroughly analyzed.

Other systems describe compositions and methods of manufacture based on fused filament extrusion techniques (FDM). For example, patent document WO2019025857 describes a filament for 3D printing comprising a substrate material containing more than 40% of a water-soluble linear polymer and an active ingredient integrated into that material. The feed for this system is a filament which must be heated above its melting temperature to generate the final pharmaceutical presentation. Patent document WO2019030109 also describes a method and apparatus for manufacturing dosage forms by additive manufacturing by means of fused filament. In both cases, the pharmaceutical forms are solid (pills) that have to be swallowed whole, which entails the drawbacks indicated above for this type of pharmaceutical form. Furthermore, there is a risk inherent to this methodology, which comprises subjecting APIs to very high temperatures, possibly resulting in a loss of activity.

Other compositions and manufacturing systems known in the state of the art are described in patent document WO2018028972. This document describes a method of manufacture which uses two fluids, with at least one of them containing an active ingredient. The two fluids are fused to form a mixture that is deposited forming a self-curing material, i.e., it undergoes a chemical process which hardens the material without the need for an external chemical additive or ultraviolet radiation, preferably rapid curing and in atmospheric conditions. These flows are generated by a head with a specific nozzle and fused in a dynamic process. This is a binder jetting process performed *in situ* and in a single step to produce solid pharmaceutical forms.

Systems for the 3D printing of medicinal products by means of semisolid extrusion (SSE), such as solid poly-pills with different APIs in each one, which seek to modify the release profile of the drug, have also been described. The medicinal products are printed by means of pastes requiring drying times to completely solidify; namely, they must be kept in a vacuum dryer at 40 °C for 24 hours until the solvent is removed (Khaled SA et al., 2015. Int J Pharm 494, 643-50). This drying period may compromise the stability of some APIs, in addition to delaying the patient taking the medication and requiring longer dosage preparation times.

GOYANES ALVARO ET AL (INTERNATIONAL JOURNAL OF PHARMACEUTICS, ELSEVIER, vol. 567, 4 July 2019) discloses personalised chewable formulations. 3D cylindrical printlets are prepared using an adapted 3D printer. The ink comprises the active agent and excipients.

SCOUTARIS NICOLAOS ET AL (PHARMACEUTICAL RESEARCH, vol. 35, no. 2, 16 January 2018, pages 1-11) describes a study using Fusion Deposition Modelling (FDM) to print candy-like formulation to prepare paediatric medicines with enhanced palatability. The ink used in this study is based on Hypromellose acetate succinate.

All the compositions and methods for the 3D manufacture of medicinal products known in the state of the art allow pharmaceutical products to be manufactured in solid dosage forms.

Therefore, there is a need to find a pharmaceutical form which allows active ingredients to be dosed in a personalized manner without compromising the activity of the active ingredient they contain, with safety (i.e., no traces of resins or materials with a potential toxicity or the toxicity of which has not been tested), and suitable for people who are unable or unwilling to swallow solid pharmaceutical forms.

### DESCRIPTION OF THE FIGURES

**Figure 1****.** Working diagram of an extrusion system of a 3D printer. The numbers corresponding with each element of the system are indicated in parentheses. The head moves on three axes (X, Y, Z) to print the figure.
**Figure 2****.** Figure printed with printing ink according to Formula B.
**Figure 3****.** Printed pharmaceutical forms. A. Percentage of infill density 65 %. B.
Percentage of infill density 80 %.
**Figure 4****.** Release profile of a pharmaceutical dosage comprising ranitidine. At least 80 % of the drug is released in 10 minutes.

### DESCRIPTION OF THE INVENTION

The invention is defined by the claims. Any subject-matter falling outside the scope of the claims is provided for information purposes only.

The solution to the problems detected in the state of the art is an ink for 3D printing, whose composition produces hydrogel-type semisolid pharmaceutical forms once printed. These pharmaceutical forms are characterized by being chewable and having doses and forms customized for each patient.

A semisolid pharmaceutical form or hydrogel refers to an object having a viscosity and rigidity midway between a solid element and liquid element. In a preferred embodiment, the semisolid form is suitable for intake and allows being swallowed whole, being cut up into pieces or being chewed effortlessly with the gums or teeth. A hydrogel (HG) is a cross-linked polymeric material of natural or synthetic origin in the form of three-dimensional network which, when contacted with water, swells and forms elastic, soft and flexible composites (Reyes Ortega F et al., 2012. Biomecanica 20; 7-19).

In one embodiment, the present invention relates a 3D printing ink, comprising an active pharmaceutical ingredient, at least three hydrocolloids and purified water, characterized in that the three hydrocolloids are gelatin, iota-carrageenan and xanthan gum. The at least three hydrocolloids are essential excipients of the composition. In a preferred embodiment, the composition may comprise auxiliary excipients that improve the organoleptic characteristics and palatability of the pharmaceutical composition. In a more preferred embodiment, the auxiliary excipients are selected from the group comprising corn starch, a sweetener, an essence and a food dye, or a combination thereof. The 3D printing ink described herein can be considered a pharmaceutical composition.

In a preferred embodiment, the one or more active ingredients (API) of the 3D printing ink are selected from one or more active ingredients that are water-soluble or in suspension.

In a still more preferred embodiment, the active ingredients are selected, without thus limiting the scope of the invention, from the group comprising the water-soluble APIs ranitidine hydrochloride, flecainide acetate, enalapril maleate, n-acetylcysteine, paracetamol, cetirizine dihydrochloride, digoxin, gentamicin sulfate or neomycin sulfate. In a more preferred embodiment, the API is ranitidine hydrochloride.

Ranitidine hydrochloride or ranitidine HCl (Drugbank accession number DBSALT000487) is an active ingredient that antagonizes histamine H₂ receptors of the parietal cells of the stomach, inhibiting the stimulated and basal secretion of gastric acid and reducing the production of pepsin. This compound is indicated in the treatment of duodenal ulcer, benign gastric ulcer, Zollinger-Ellison syndrome, prevention of recurrent hemorrhage in patients with a bleeding ulcer, peptic esophagitis and treatment of associated symptoms, prevention of gastrointestinal hemorrhage due to stress ulcers, in patients with a risk of acid aspiration (Mendelson's syndrome) or in obstetric patients during childbirth. In children it is used as a short-term treatment of peptic ulcer and in the treatment of gastro-esophageal reflux. Ranitidine is marketed in the form of tablets which must be taken whole with water and cannot be broken up or crushed (Source: Vademecum). In a preferred embodiment, the ranitidine HCl is used at a dose between 30 mg and 600 mg, based on the age and weight of the patient and on the pathology to be treated. In a preferred embodiment, the concentration of ranitidine HCl in the ink is between 3 % and 15 % with respect to the total weight of the ink (w/w). The dose will depend on the patient to be treated (for example, adults or children). In a more preferred embodiment, the ranitidine HCl is at a concentration between 3.3 % and 3.5 % with respect to the total weight of the ink. This preferred concentration is targeted at the pediatric population.

Iota-carrageenan (i-carrageenan) is a gelling agent formed by chains with alternating units of galactose with a sulfate group at carbon 4 and anhydrogalactose with a sulfate group at carbon 2, joined by alternating α(1-3) and β(1-4) bonds. They are found in nature filling in gaps in the cellulose structure of cell walls in some red algae, such as for example *Chondrus crispus* or algae of the genus *Eucheuma.* In order to be used as a gelling agent, the chains must have a weight molecular from 100,000 Da. Carrageenans are soluble in hot conditions, from 70 °C, and remain in solution upon cooling if they are in sodium salt form. Carrageenan gels are thermally reversible. I-carrageenan forms gels in the presence of calcium ions, gels that are elastic, strong, without a tendency for syneresis and which withstand freezing. Furthermore, this type of carrageenan improves the properties of starch gels. In a preferred embodiment, i-carrageenan is used at a concentration greater than 2 % w/w with respect to the total weight of the ink. An ink with an iota-carrageenan content less than 2 % does not reproduce the desired structure with fidelity or maintain the shape when printed. In a more preferred embodiment, the composition comprises i-carrageenan at a concentration between 2 % and 4 % (w/w).

Xanthan gum is an extracellular polysaccharide produced by the bacterium *Xanthomonas campestris B*-1459 used as a thickener due to its stability in a wide range of pH, salt concentration and temperatures. In a preferred embodiment, xanthan gum is used at a concentration of 0.25 - 0.50 % w/w with respect to the total weight of the ink. Neither the necessary elasticity nor the semisolid texture of the composition of the invention at room temperature is obtained below a concentration of 0.25 %. Values above 0.50 % hinder the removal of bubbles from the formula, which is counterproductive because it affects the final weight of the figure and, therefore, the dosage of the medicinal product.

Gelatin is a product derived from animal collagen, which is soluble in water. In a preferred embodiment, the concentration of gelatin in the 3D printing ink is between 5 and 8 % w/w with respect to the total weight of the ink. Values of less than 5 % may cause problems of syneresis in the composition (phase separation). Values closer to 8 % cause the compositions to be more readily manipulated. In a more preferred embodiment, the composition comprises type A gelatin.

A liquid sweetener is any natural or artificial substance that serves for imparting a sweet flavor to a food or product which otherwise has a bitter or unpleasant flavor. Any sweetener suitable for foods in liquid form can be used, such as for example, brazzein, curculin, erythritol, fructose, glycyrrhizin, glycerol, hydrogenated starch hydrolysates, lactitol, luo han guo, mabinlin, maltitol, maltooligosaccharides, mannitol, miraculin, monellin, pentadin, sorbitol, Stevia, tagatose, thaumatin, xylitol, acesulfame K, advantame, alitame, aspartame, cyclamate, dulcin, glucin, isomalt, neohesperidin hydrochalcone, neotame, P-4000, saccharine, aspartame-acesulfame salt, sucralose or inulin, among others. In a preferred embodiment, the sweetener is selected from cyclamate and saccharine. In a preferred embodiment, the liquid sweetener is used at a concentration between 0 and 3.5 % w/w with respect to the total weight of the ink.

For purposes of the present invention, the essence is any food additive that improves the organoleptic characteristics of a product to be ingested. In a preferred embodiment, the essence can be of a natural or artificial origin at a concentration between 0.1 - 0.5 % w/w with respect to the total weight of the ink. As an example, but without limiting the invention, the essence can be selected from the group comprising strawberry essence, orange essence, vanilla essence, lemon essence, mint essence, among others. In a more preferred embodiment, the essence is strawberry essence.

Food dyes are a type of food additive that imparts color to foods. The food dye can be selected from any innocuous dye. As an example, but without limiting the invention, the food dye is selected from the group comprising curcumin, riboflavin, lactoflavin, lactoflavin phosphate, indigotin, chlorophylls A and B, chlorophyll copper complexes and chlorophyllins, carotenoids, xanthophylls, betanin, anthocyanins, calcium carbonate or iron oxide. In a preferred embodiment, the food dye is used at a concentration between 0 and 0.5 % w/w with respect to the total weight of the ink.

The purified water is drinking water that has been subjected to a purification process to enable meeting quality standards and being suitable for use in pharmacies and laboratories. Furthermore, it complies with international standards relating to water, such as ISO 3696. This water is free of chlorine and other chemical substances. The systems of purification can be by means of filtering, reverse osmosis or any system known by a person skilled in the art.

Corn starch is a macromolecule made up of two polysaccharides: amylose (25 %) and amylopectin (75 %). The starch is used as an auxiliary excipient to improve the texture and palatability of the formula. In a preferred embodiment, the concentration of corn starch in the composition is comprised between 5 and 8 % w/w with respect to the total weight of the ink. In a still more preferred embodiment, the concentration of corn starch is 5 % w/w. Values greater than 8 % of starch cause the figure printed by 3D technology to lose fidelity with respect to the desired design, as the viscosity of the printed product increases too much, while a concentration less than 5 % produces figures with a less pleasant taste.

In a preferred embodiment, the 3D printing ink comprises a concentration between 3.0 % and 15.0 % w/w of ranitidine hydrochloride, 2 - 4 % w/w of iota-carrageenan, 0.25 - 0.50 % w/w of xanthan gum, 5 - 8 % w/w of type A gelatin, 0 - 3.5 % w/w excluding 0 of liquid sweetener 0.1 - 0.5 % of strawberry essence, 0 - 0.5 % excluding 0 of food dye and purified water. In a more preferred embodiment, the composition further comprises 5 - 8 % w/w of corn starch. All the percentages of the ingredients are with respect to the total weight of the ink.

The composition of the invention has the characteristic of being in a liquid state at a temperature comprised between 30 and 40 °C and solidifying when their temperature is below 25 °C. Due to the hydrocolloids present in its composition, at room temperature this composition is in a semisolid state. Semisolid state refers to a composition having a viscosity and rigidity midway between a solid material and a liquid material. In the present invention, the semisolid consistency is closer to that of a solid material, given that it is stable, maintains its shape and has a gummy structure. This structure has the advantage that the composition is chewable, which improves the swallowing of the medicinal product, particularly in the groups of patients who have the greatest problems when swallowing, such as pediatric and geriatric patients.

In another embodiment, the invention relates to a method for preparing a pharmaceutical composition in the form of 3D printing ink as defined above, comprising the following steps:
a) contacting 5 - 8 % w/w of gelatin and 5-7 times of purified water with respect to the weight of the gelatin, at room temperature for 10 - 20 minutes to obtain a hydrated gelatin;
b) heating the hydrated gelatin of step a) at 40 ± 2 °C;
c) adding a solution of active pharmaceutical ingredient (API) in purified water at room temperature to a mixture of i-carrageenan at 2 - 4 % w/w and xanthan gum at 0.25 - 0.50 % w/w, both percentages with respect to the total weight of the ink, in purified water at 35 - 40 °C and stirring to obtain a homogeneous mixture;
d) adding the hydrated gelatin of step b) to the homogeneous mixture of step c) to obtain a second mixture;
e) adding to the second mixture of step d) 0.1 - 0.5 % w/w of an essence, 0 - 3.5 % w/w of sweetener excluding 0, 0 - 0.5 % w/w of food dye excluding 0, where the percentages are with respect to the total weight of the ink, and gently mixing;
f) incubating for 30 - 60 minutes at 40 - 45 °C to obtain the printing ink.

In step a), the gelatin is added to a volume of water between 5 and 7 times its weight. In a preferred embodiment, a volume of purified cold water 6 times the weight of the gelatin is added. In a preferred embodiment, the gelatin in step a) is type A gelatin. The hydration of the gelatin in said step takes place at room temperature. In step b), the increase in temperature causes the hydrated gelatin to melt.

The active ingredients that are part of the pharmaceutical composition must be dissolved in purified water in step c). In a preferred embodiment, the API is ranitidine hydrochloride. In a more preferred embodiment, the concentration of ranitidine hydrochloride is 3 - 15 % (w/w). In a still more preferred embodiment, the concentration of ranitidine hydrochloride is 3.3 - 3.5 % (w/w) for subjects from 5 years of age. Optionally, the method may include an additional step in which starch is added to the solution of the API as an auxiliary excipient. In a preferred embodiment, the starch is corn starch, preferably at a concentration of 5 - 8 % w/w with respect to the total weight of the ink. In this case, the mixture of the solution of the API and starch must be stirred constantly and non-vigorously until use so that the starch does not precipitate, and the suspension is homogeneous. The i-carrageenan and xanthan gum are then dissolved together in purified water at 35 - 40 °C. The dissolved active ingredient/active ingredients are gradually added to this mixture. The mixture is stirred until it is homogeneous and forms a paste.

In step d), the hydrated and melted gelatin of step b) is added to the mixture of step c). It is important that the addition of the gelatin takes place under continuous stirring for the ingredients to be combined homogeneously, but it must be gentle to avoid the formation of bubbles or foam which subsequently affects the texture of the composition.

The rest of the excipients (essence, food dye and sweetener) are then added to the mixture in any order and are gently mixed until the obtained mixture is homogeneous (step e)). Lastly, in step f), stirring is removed and the mixture is incubated for 30 - 60 minutes at 40 - 45 °C until there are no bubbles.

The present disclosure also describes the use of the 3D printing ink as defined above, or the composition obtained from the described method, as ink for a 3D printer by semisolid extrusion.

The present disclosure also describes ink for 3D printing by semisolid extrusion comprising: 2 - 4 % w/w iota-carrageenan (i-carrageenan), 0.25 - 0.50 % w/w xanthan gum, 5 - 8 % w/w gelatin, 0 - 3.5 % excluding 0 of liquid sweetener, 0.1 - 0.5 w/w % essence, 0 - 0.5 % w/w excluding 0 of food dye and purified water. In a preferred embodiment, the printing ink additionally comprises 5 - 8 % w/w of corn starch. All the percentages of the ingredients are with respect to the total weight of the ink.

Another embodiment of the invention relates to a method for manufacturing a pharmaceutical form, characterized in that it comprises the following steps:
a) filling a first cartridge with the 3D printing ink according to one of the preceding embodiments, comprising a first active ingredient at a temperature between 34 - 40 °C;
b) introducing the cartridge of step a) in the extruder of a 3D printer;
c) setting the temperature of the extruder of the printer at 35 - 37 °C and the temperature of a print bed at 15 - 19 °C;
d) printing the pharmaceutical form.

In a more preferred embodiment, the invention relates to a method for manufacturing a pharmaceutical form, characterized in that it comprises the following additional steps:
e) filling a second cartridge with the 3D printing ink with a second active ingredient according to one of the preceding embodiments at a temperature between 34 - 40 °C;
f) introducing the cartridge of step e) in the extruder of a 3D printer;
g) setting the temperature of the extruder of the printer at 35 - 37 °C and the temperature of a print bed at 15-19° C;
h) printing the second part of the pharmaceutical form.

This method allows pharmaceutical forms comprising more than one active ingredient in one same dosage unit to be obtained. To that end, two 3D printing inks according to the preceding embodiments are formulated separately: ink 1 with API 1, ink 2 with API 2 and so on and so forth. At the time of the printing, the cartridges are loaded in the printer (each one with a different ink, based on the API number of the dosage), each cartridge in its extruder. After printing, a figure is generated which corresponds with a pharmaceutical form comprising the desired APIs, but each in a different composition, without them being mixed together in the printed figure. The printing of each API in one same figure may take place in multiple ways: a layer of each ink, the center of the figure of a first ink 1 and a coating of a second ink 2, etc.

The cartridges in step a) cannot have air in them in order to avoid defects in the figure. Before the printing method, the cartridge loaded with the composition can be kept between 34 and 40 ±2 °C in a bath, in order to reach a homogeneous temperature in the entire cartridge and thus enable printing at the temperature at which the extruder of the printer is configured. While the cartridges are not being used, they can be kept at 4 - 8 °C.

The present disclosure also describes pharmaceutical forms obtained from the preceding methods. In a preferred embodiment, the pharmaceutical form comprises 2 - 4 % w/w iota-carrageenan (i-carrageenan), 0.25 - 0.50 % w/w xanthan gum, 5 - 8 % w/w gelatin, 0 - 3.5 % excluding 0 of liquid sweetener, 0.1 - 0.5 w/w % essence, 0 - 0.5 % w/w excluding 0 of food dye and purified water. In a more preferred embodiment, the figure also comprises 5 - 8 % w/w of corn starch. All the percentages of the ingredients are with respect to the total weight of the pharmaceutical form.

The specific dose of the API or APIs present in the printed pharmaceutical form (or dosage unit) will depend on the needs of each patient. The concentration of the API or APIs in the ink will vary based on whether said pharmaceutical form (or dosage unit) will be administered to pediatric patients or adults. The concentration of the one or more active ingredients in the ink does not determine the pharmaceutical dose to be administered in a patient. What determines the dose is the final printed form.

3D printing requires a prior design which can be done by means of a modelling program (CAD, computer-aided design, type program) to create a three-dimensional representation of the object to be printed. The modelling program generates a file which can be an STL (STereoLithography), VRML (virtual reality modelling language) or AMF (additive manufacturing format) type, among others. In the case of printing several APIs in one same figure, thorough knowledge of these programs is required, given the design of figures which comprise two or more materials and require printers with more than one extruder to enable working efficiently, as it is inviable to change the cartridge every time part of the figure is changed. In a preferred embodiment, the design of the object is saved in STL format. The cutting or slicing process is then performed by means of an external program (for example, slic3r or cura 3D among others) or a proprietary program of the 3D printer. This process is automatic and involves cutting the figure into several successive layers and recording the information contained in each layer so that the printer can subsequently execute the corresponding paths. In this step, it is possible to set the printing parameters which will condition the appearance of the resulting figure (weight, density, strength, etc.).

Another embodiment of the invention relates to the pharmaceutical form obtained in the methods of the preceding embodiments for use in the treatment of a disease.

Another embodiment of the invention relates to the pharmaceutical form obtained in the methods of the preceding embodiments for use in the treatment of duodenal ulcer, benign gastric ulcer, Zollinger-Ellison syndrome, prevention of recurrent hemorrhage in patients with a bleeding ulcer, peptic esophagitis, prevention of gastrointestinal hemorrhage, treatment of patients with a risk of acid aspiration or obstetric patients during childbirth in adults.

In another embodiment, the pharmaceutical form obtained in the methods of the preceding embodiments is for use in the treatment of peptic ulcer and in the treatment of gastro-esophageal reflux in pediatrics.

In another embodiment, the dosage obtained in the methods of the preceding embodiments is for use in oral or sublingual administration to a patient.

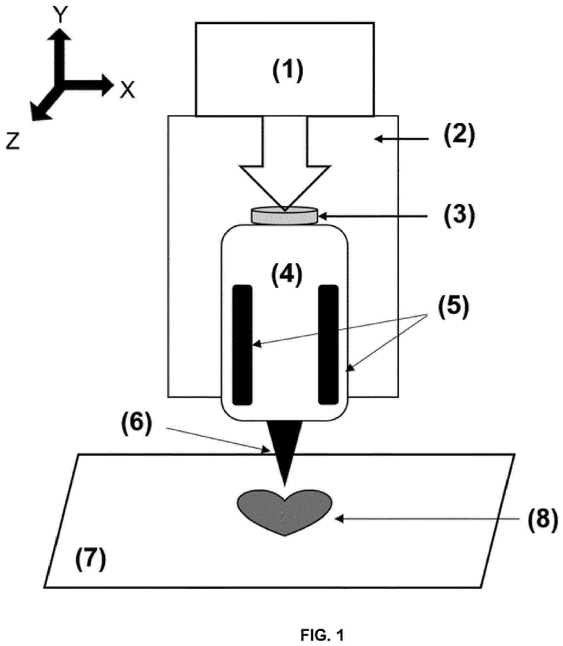

**Figure 1** shows an extrusion system (1) of a 3D printer. The 3D printing ink of the invention (or pharmaceutical composition) present in the container cartridge (3) is thermally reversible, i.e., it goes from liquid to solid upon cooling. This property is utilized for the pharmaceutical composition to be used as a printing ink, which flows while hot through the extruder (4) of the head (2) of the printer and is deposited on the print bed (7) where it is cooled and recovers rigidity to withstand the weight of the next layers of the figure. The temperature regulation system of the extruder (5) allows selecting the temperature at which the printing ink exits the nozzle (6). The printed figure (8) solidifies in the print bed (7) as layers of ink are deposited on the bed.

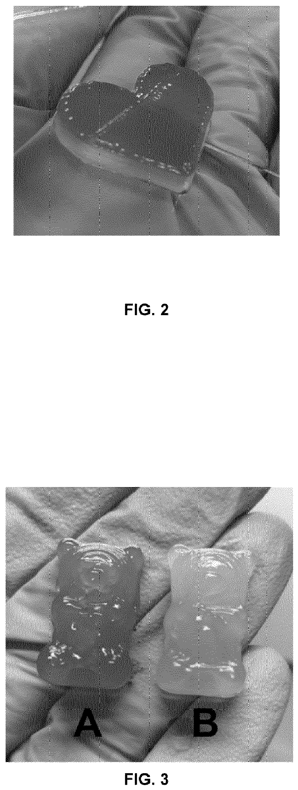

The 3D printing ink of the invention allows producing semisolid pharmaceutical forms with a personalized dosing of one or more active ingredients customized for each patient by means of varying the manufacturing and/or design parameters of the figure. 3D printing technology produces medicinal products with a precise API dose based on its initial amount in the ink, but if furthermore depends on the physical dimensions of the figure to be printed. A personalized dosing of the API is thus achieved as it is possible to modify the printed figure both in size (larger size, higher dose) and in percentage of filler thereof (for one same size, a figure with a higher percentage of filler contains a higher dose). As a result of 3D printing, it is possible to select any shape of the medicinal product printed in the software associated with the printer, which allows, for example, more appealing and/or smaller designs for pediatric patients, instead of the usual rigid and hard-to-swallow solid forms.

The figures (pharmaceutical forms) obtained from the 3D printing ink, printed with a 3D printer have a weight in accordance with the type of chewable dosage unit to be obtained (generally 0.50 g and 2.00 g). Logically, the dose for a normal adult will be much higher than the dose for a child, and for that reason an adult will take a pill that has a higher weight (larger amount of ink) and a child will take a smaller one. By way of illustration and without limiting the invention, a formulation comprising 1.004 mg of ranitidine HCl for every 30 g of ink is indicated for a child about 5 years old to take two 0.80 - 0.90 g pills. If this same child were to have a low weight and needed a smaller dose, figures having 0.50 - 0.60 g could be made, and if the child were suffering a relapse or an acute phase of a pathology, pharmaceutical forms having up to 2.00 g could be produced to meet that child's requirements. The pharmaceutical forms of the present invention allow the concentration of each active ingredient to tailor to the specific needs of each patient, allowing a personalized treatment.

The advantages of the medicinal products (pharmaceutical forms) obtained by means of 3D printing technology described above are:
- Increase in adherence to treatment and acceptance by the patient (especially in pediatric patients). Taking several medicinal products, a day is difficult for children taking multiple medicines, especially when taking solid oral pharmaceutical forms. The possibility of administering more than one drug at a time in a hydrogel and with a shape that is appealing to them would lessen the psychological and social impact of the disease while at the same time causing the acceptance of treatment to substantially increase.
- Improved quality of life of patients with dysphagia. Taking a hydrogel directly is much more comfortable and simpler for a patient with this type of limitation. Furthermore, processes such as the undue opening of hard capsules or the manual grinding of tablets which, in addition to considerably jeopardizing the formulation of the medicinal product (Parodi et al., 2015. Journal of the American College of Cardiology, 65: 511-512), is difficult in most cases, are avoided.
- Possibility of enhancing the sublingual administration route: The sublingual route has pharmacokinetic advantages over the oral route. The main advantage is the fast absorption process, with plasma concentrations of active ingredient being obtained 3 minutes after administration (Blumenthal et al., 1977 Br J Clin Pharmacol. 1977 (2):241-2; Marseglia et al., 2009 Ital J Pediatr. 2009 35(1):31), becoming an ideal route for the treatment of emergency conditions such as angina pectoris, anxiety or acute pain. It must also be taken into account that this route has no hepatic first-pass effect, so the possible alteration of the drug or its gastrointestinal or hepatic inactivation is avoided, and it is a potential path of study for APIs which, because of this metabolic phenomenon, must be administered through other routes that are more difficult to use, such as intravenous or intramuscular routes.
- Possibility of compensating for nutritional deficiencies or other shortages while at the same time medicating the patient. Specific vitamin deficiencies or low trace element levels, for example, could be treated while at the same time a drug could be administered for a specific condition.

### EXAMPLES

### Example 1: Preparation of the composition

**Table 1: Components of the composition**

| **Component** | **Trade name** | **Supplier** | **Batch** |
|---|---|---|---|
| Ranitidine hydrochloride | - | Fagron Iberica SAU | 18F08-B01-352568 |
| Iota-carrageenan | Gelificación Iota | Texturas^{®} | L6329 |
| Xanthan gum | - | Fagron Iberica SAU | L18030182 |
| Gelatin from porcine skin | - | SIGMA - ALDRICH | #SLBT2992 |
| Corn starch | Maizena^{®} | Unilever | L81970M776 |
| Liquid sweetener | Ciclamato y Sacarina sódica para endulzar | Hacendado | 28D11/30358 |
| Strawberry essence | Esencia alimenticia de fresa | Fagron Iberica SAU | 18H01-H17-00714 |
| Food dye | Vahiné Colorantes Alimentarios | McCormick España, S.A. | F062 |
| Purified water | - | Fagron Iberica SAU | 18G18-T07-058201 |

**Table 2: Ink for printing (Formula A)**

| **Components** | **Weight (g)** | **[c] (% w/w)** |
|---|---|---|
| Ranitidine HCl | 1.004 | 3.35 |
| Iota-carrageenan | 0.600 | 2.00 |
| Xanthan gum | 0.075 | 0.25 |
| Gelatin type A | 2.400 | 8.00 |
| Corn starch | 1.500 | 5.00 |
| Liquid sweetener | 1.000 | 3.33 |
| Strawberry essence | 0.150 | 0.50 |
| Food dye | 0.150 | 0.50 |
| Purified water | q.s. | |
| Total | 30.000 | |

**Table 3: Ink for printing (Formula B)**

| **Components** | **Weight (g)** | **[c] t (% w/w)** |
|---|---|---|
| Ranitidine HCl | 1.004 | 3.35 |
| Iota-carrageenan | 0.600 | 2.00 |
| Xanthan gum | 0.075 | 0.25 |
| Gelatin type A | 2.400 | 8.00 |
| Liquid sweetener | 1.000 | 3.33 |
| Strawberry essence | 0.150 | 0.50 |
| Food dye | 0.150 | 0.50 |
| Purified water | q.s. | |
| Total | 30.000 | |

For preparing both formulas, 2.4 g of type A gelatin powder were weighed in a vessel and 15 g of purified water at room temperature were added, trying to cover all the powder. The gelatin was left to hydrate for more than 15 minutes and the vessel with the hydrated gelatin was introduced in a hot bath (40 °C approximately) until the gelatin was melted (turned completely liquid).

The API (ranitidine hydrochloride) was then weighed and dissolved in about 4 g of purified water.

In the case of formula A, 1.500 g of corn starch were weighed and added to the API solution. The mixture was stirred until the solution was homogeneous.

0.600 g of i-carrageenan and 0.075 g of xanthan gum were weighed in another vessel. About 4 g of purified warm water (35 - 40 °C) were added to facilitate the homogeneous mixing of components. The starch and API suspension were added manually and by gently stirring the mixture of i-carrageenan and xanthan gum. The mixture was mixed until it was homogeneous, forming a dense paste. It is important for this mixing to be slow to avoid the incorporation of air.

The hydrated gelatin was then added little by little and fused to the previous mixture under continuous but gentle stirring to avoid foaming.

1 g of liquid sweetener, 0.150 g of strawberry essence and 0.150 g of food dye were weighed in one same vessel and added to the previous mixture. The composition was gently stirred until a homogeneous mixture was obtained. Once homogenized, it was left to stand in a bath at 40 - 45 °C for 30 - 60 minutes until there was no bubble generated by the stirring of the mixture.

For preparing Formula B, the method was identical to the one used for Formula A, but without starch in its composition.

### Example 2. Method of 3D printing of a medicinal product

The medicinal product (or pharmaceutical form) was printed using a mechanical extrusion printer manufactured in-house with temperature control in the head and in the print bed. The printer used a custom-developed control software.

First, the 3D model of the figure to be printed was loaded in the Slic3r slicing software. The software settings determine the printing speed, density or filler and resolution. The slicing program generated the gcode file subsequently read and executed by the printer control software.

The cartridge containing the formulated ink (formula A or formula B) was then introduced in the extruder which was at a temperature comprised between 35 and 37 °C and the temperature of the print bed was set at 15 - 19 °C.

The printing process was started from the printer control software, acting on the extrusion mechanism to release the formulated ink onto the print bed layer by layer according to the input 3D model and the slicing instructions. The layer of extruded material was cooled on contact with the print bed of the printer, transitioning to a solid state. This step was repeated the necessary times until the input 3D model was generated. Once printing ended, the printed figure was removed and kept in a refrigerator (4 - 8 °C) in a clean and dry vessel. Figures printed with this ink can be kept for more than 24 hours without losing their shape or their texture at room temperature. They can be kept for much longer if they are kept refrigerated, remaining intact for weeks.

**Figure 2** shows a figure printed by means of the preceding method. The figure is formulated according to the printing ink of Formula B.

### Example 3. Strategies for personalizing the dose of an active ingredient

### 3.1 Scale of the medicinal product

3D printing allows figures on a different scale to be generated from one same design or model. A higher or lower dosage of an active ingredient is thereby achieved. The larger the size, the greater the weight and the higher the dose will be. The scaling process is performed by means of the same slicing program or any control software of the printer itself.

### 3.2 Percentage of filler of the figure printed

The dose of an active ingredient can be modified maintaining the size of a figure, i.e., at the same scale. The difference is the internal structural filler of the printed figure. The more filler there is, the larger the amount of ink, the greater the weight of the figure and the higher the dose of API there will be.

Figure 3 shows two figures having the same size but comprising different concentrations of active ingredient. Both figures have the composition described in Formula B of Example 1. Figure A has 65 % filler, whereas figure B has 80 %. The percentage of filler of a piece is a parameter that defines the solidity of the printed figure: the higher the percentage of filler, the greater the density and, therefore, the higher the dose. An ink having for example 3.5 % of API in a pharmaceutical form with 65 % of filler will contain a dose of 39 mg of said API, whereas in the case of 80 % of filler, the final dose of the API will be 45 mg. This allows the shape of the printed figures to be modified in order to have pharmaceutical forms with the suitable patient-based dose of an API. The modification of the mass of the pharmaceutical form allows the dose present therein to be modified.

### Example 4: Dissolution test and drug release profiles

Dissolution studies are commonly used to simulate the *in vitro* behavior of pharmaceutical doses and predict their bioavailability and efficacy *in vivo.*

Drug release profile of the medical product printed by the method of Example 2 was determined with an USP-II apparatus (Erweka D700, Germany). It was used the dissolution method of the USP monograph for ranitidine tablets (USP 43-NF 38), since a specific test for chewable doses has not been established yet. For this type of assay, USP tolerances data establish that not less than 80% of the contained amount of ranitidine must be dissolved within 45 minutes.

Each product analyzed, which contained approximately 30 mg of ranitidine, was placed in a container with purified water as dissolution medium (900 mL), kept at 37 ° C ± 0.5 ° C with stirring at 50 rpm. Samples were taken at 5, 10, 15, 30, 45, 60, 90 and 120 minutes and the amount of dissolved ranitidine was determined for each sample by uv-vis spectrophotometry (Specord 205, Analytical Jena, Germany). Six printed dosages were tested. Food coloring was not included in the ink to not interfere with the spectrophotometry technique and therefore ensure adequate ranitidine quantification.

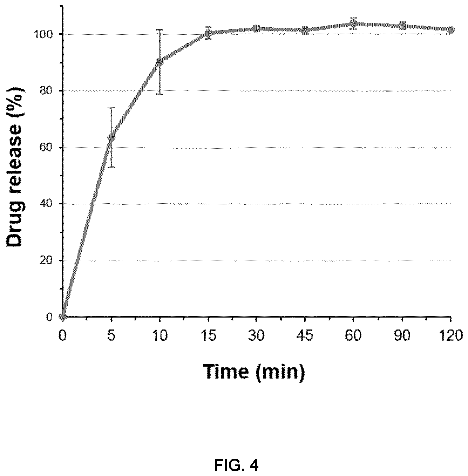

As can be seen in **Figure 4**, the resulting dissolution profile of the pharmaceutical form shows that more of the 80 % of the API was released after 10 minutes. This is an excellent result, since the Pharmacopeia assay requires at least 80 % of release in less than 45 minutes.

## Claims

1. A composition comprising an active pharmaceutical ingredient, at least three hydrocolloids and purified water, **characterized in that** the three hydrocolloids are gelatin, iota-carrageenan and xanthan gum, wherein the composition is a 3D printing ink.

2. The composition according to claim 1, **characterized in that** the composition additionally comprises an excipient selected from the group consisting of corn starch, sweetener, an essence and a food dye, or a combination thereof.

3. The composition according to one of claims 1 or 2, **characterized in that** the active pharmaceutical ingredient is a water-soluble active ingredient or an active ingredient in suspension.

4. The composition according to claim 3, **characterized in that** the water-soluble active ingredient is selected from the group consisting of ranitidine hydrochloride, flecainide acetate, enalapril maleate, n-acetylcysteine, paracetamol, cetirizine dihydrochloride, digoxin, gentamicin sulfate and neomycin sulfate.

5. The composition according to one of claims 1 to 4, **characterized in that** the ink comprises 2 - 4 % w/w of iota-carrageenan with respect to the total weight of the ink.

6. The composition according to one of claims 1 to 5, **characterized in that** the composition comprises 0.25 - 0.50 % w/w of xanthan gum with respect to the total weight of the composition.

7. The composition according to one of claims 1 to 6, **characterized in that** the composition comprises 5 - 8 % w/w of gelatin with respect to the total weight of the composition.

8. The composition according to one of claims 2 to 7, **characterized in that** the composition comprises between 5 and 8 % w/w of corn starch with respect to the total weight of the composition.

9. The composition according to one of claims 1 to 8, **characterized in that** the active pharmaceutical ingredient is ranitidine hydrochloride.

10. The composition according to claims 1 to 9, **characterized in that** the composition comprises 3 - 15 % of ranitidine hydrochloride, 2 % - 4 % w/w of iota-carrageenan, 0.25 - 0.50 % w/w of xanthan gum, 5 - 8 % w/w of type A gelatin, 0 - 3.5 % w/w of a liquid sweetener excluding 0, 0.1 - 0.5 % w/w of strawberry essence, 0 - 0.5 % excluding 0 of a food dye, 5 - 8 % w/w of corn starch and purified water, where the percentages are with respect to the total weight of the composition.

11. A method for preparing a pharmaceutical composition in the form of 3D printing ink, **characterized in that** it comprises the following steps:
a) contacting 5 - 8 % w/w of a gelatin and 5-7 times of purified water with respect to the weight of the gelatin, at room temperature for 10 - 20 minutes to obtain a hydrated gelatin;
b) heating the hydrated gelatin of step a) at 40 ± 2 °C;
c) adding a solution of active pharmaceutical ingredient (API) in purified water at room temperature to a solution of i-carrageenan at 2 - 4 % w/w and xanthan gum at 0.25 - 0.50 % w/w, both percentages with respect to the total weight of the ink, in purified water at 35 - 40 °C and stirring to obtain a homogeneous mixture;
d) adding the hydrated gelatin of step b) to the homogeneous mixture of step c) to obtain a second mixture;
e) adding to the second mixture of step d) 0.1 - 0.5 % w/w of an essence, 0 - 3.5% w/w excluding 0 of a sweetener, 0 - 0.5 % w/w excluding 0 of a food dye, where the percentages are with respect to the total weight of the ink, and gently mixing;
f) incubating for 30 - 60 minutes at 40 - 45 °C to obtain the printing ink.

12. The method according to claim 11, **characterized in that** 5 - 8 % w/w of corn starch with respect to the total weight of the ink is added in step c).

13. The method according to claims 11 and 12, **characterized in that** the active ingredient in step c) is ranitidine hydrochloride.

14. A method for manufacturing a pharmaceutical form **characterized in that** it comprises the steps of:
a) filling a first cartridge with the 3D printing ink according to one of claims 1 to 10 comprising a first active ingredient at a temperature between 34 - 40 °C;
b) introducing the cartridge of step a) in the extruder of a 3D printer;
c) setting the temperature of the extruder of the printer at 35 - 37 °C and the temperature of a print bed at 15 - 19 °C;
d) printing the pharmaceutical form.

15. The method for manufacturing a pharmaceutical form according to claim 14, **characterized in that** it comprises the additional steps of:
e) filling a second cartridge with the 3D printing ink according to one of claims 1 to 10 with a second active ingredient at a temperature between 34 - 40 °C,
f) introducing the cartridge of step e) in the extruder of a 3D printer;
g) setting the temperature of the extruder of the printer at 35 - 37 °C and the temperature of a print bed at 15 - 19° C;
h) printing said pharmaceutical form.

16. The pharmaceutical form obtained in claims 14 or 15, for use in the treatment of a disease.

17. The pharmaceutical form obtained in claims 14 or 15, for use in the treatment of duodenal ulcer, benign gastric ulcer, Zollinger-Ellison syndrome, prevention of recurrent hemorrhage in patients with a bleeding ulcer, peptic esophagitis, prevention of gastrointestinal hemorrhage, treatment of patients with a risk of acid aspiration or obstetric patients during childbirth in adults.

18. The pharmaceutical form obtained in claims 14 or 15, for use in the treatment of peptic ulcer and in the treatment of gastro-esophageal reflux in pediatrics.

## Patentansprüche

1. Zusammensetzung, die einen pharmazeutischen Wirkstoff, mindestens drei Hydrokolloide und gereinigtes Wasser umfasst, **dadurch gekennzeichnet, dass** die drei Hydrokolloide Gelatine, lota-Carrageen und Xanthan sind, wobei die Zusammensetzung eine 3D-Druckfarbe ist.

2. Zusammensetzung nach Anspruch 1, **dadurch gekennzeichnet, dass** die Zusammensetzung zusätzlich einen Hilfsstoff umfasst, der aus der Gruppe ausgewählt wird, die aus Maisstärke, Süßstoff, einer Essenz und einem Lebensmittelfarbstoff oder einer Kombination davon besteht.

3. Zusammensetzung nach einem der Ansprüche 1 oder 2, **dadurch gekennzeichnet, dass** der pharmazeutische Wirkstoff ein wasserlöslicher Wirkstoff oder ein Wirkstoff in Suspension ist.

4. Zusammensetzung nach Anspruch 3, **dadurch gekennzeichnet, dass** der wasserlösliche Wirkstoff aus der Gruppe ausgewählt wird, die aus Ranitidinhydrochlorid, Flecainidacetat, Enalaprilmaleat, N-Acetylcystein, Paracetamol, Cetirizindihydrochlorid, Digoxin, Gentamicinsulfat und Neomycinsulfat besteht.

5. Zusammensetzung nach einem der Ansprüche 1 bis 4, **dadurch gekennzeichnet, dass** die Druckfarbe 2 - 4 % w/w lota-Carrageen, bezogen auf das Gesamtgewicht der Druckfarbe, umfasst.

6. Zusammensetzung nach einem der Ansprüche 1 bis 5, **dadurch gekennzeichnet, dass** die Zusammensetzung 0,25 - 0,50 % w/w Xanthan, bezogen auf das Gesamtgewicht der Zusammensetzung, umfasst.

7. Zusammensetzung nach einem der Ansprüche 1 bis 6, **dadurch gekennzeichnet, dass** die Zusammensetzung 5 - 8 % w/w Gelatine, bezogen auf das Gesamtgewicht der Zusammensetzung, umfasst.

8. Zusammensetzung nach einem der Ansprüche 2 bis 7, **dadurch gekennzeichnet, dass** die Zusammensetzung zwischen 5 und 8 % w/w Maisstärke, bezogen auf das Gesamtgewicht der Zusammensetzung, umfasst.

9. Zusammensetzung nach einem der Ansprüche 1 bis 8, **dadurch gekennzeichnet, dass** der pharmazeutische Wirkstoff Ranitidinhydrochlorid ist.

10. Zusammensetzung nach einem der Ansprüche 1 bis 9, **dadurch gekennzeichnet, dass** die Zusammensetzung 3 - 15 % Ranitidinhydrochlorid, 2 % - 4 % w/w lota-Carrageen, 0,25 - 0,50 % w/w Xanthan, 5 - 8 % w/w Gelatine Typ A, 0 - 3,5 % w/w eines flüssigen Süßstoffes, ausgenommen 0, 0,1 - 0,5 % w/w Erdbeeressenz, 0 - 0,5 % eines Lebensmittelfarbstoffs, ausgenommen 0, 5 - 8 % w/w Maisstärke und gereinigtes Wasser umfasst, wobei sich die Anteile auf das Gesamtgewicht der Zusammensetzung beziehen.

11. Verfahren zum Herstellen einer pharmazeutischen Zusammensetzung in Form von 3D-Druckfarbe, **dadurch gekennzeichnet, dass** es folgende Schritte umfasst:
a) Inkontaktbringen von 5 - 8 w/w einer Gelatine und dem 5-7-fachen an gereinigtem Wasser, bezogen auf das Gewicht der Gelatine, bei Raumtemperatur für 10 - 20 Minuten, um eine hydratisierte Gelatine zu erhalten;
b) Erhitzen der hydratisierten Gelatine aus Schritt a) auf 40 ± 2 °C;
c) Zugeben einer Lösung des pharmazeutischen Wirkstoffes (API) in gereinigtem Wasser bei Raumtemperatur zu einer Lösung von I-Carrageen mit 2 - 4 % w/w und Xanthan mit 0,25 - 0,50 % w/w, beide Anteile bezogen auf das Gesamtgewicht der Druckfarbe, in gereinigtem Wasser bei 35 - 40 °C und Rühren, um ein homogenes Gemisch zu erhalten;
d) Zugeben der hydratisierten Gelatine aus Schritt b) zu dem homogenen Gemisch aus Schritt c), um ein zweites Gemisch zu erhalten;
e) Zugeben von 0,1 - 0,5 % w/w einer Essenz, 0 - 3,5 % w/w eines Süßstoffes, ausgenommen 0, 0 - 0,5 % w/w eines Lebensmittelfarbstoffes, ausgenommen 0, zu dem zweiten Gemisch aus Schritt d), wobei sich die Anteile auf das Gesamtgewicht der Druckfarbe beziehen, und sorgfältiges Mischen;
f) Inkubieren für 30 - 60 Minuten bei 40 - 45 °C, um die Druckfarbe zu erhalten.

12. Verfahren nach Anspruch 11, **dadurch gekennzeichnet, dass** in Schritt c) 5 - 8 % w/w Maisstärke, bezogen auf das Gesamtgewicht der Druckfarbe, zugegeben wird.

13. Verfahren nach Anspruch 11 und 12, **dadurch gekennzeichnet, dass** der Wirkstoff in Schritt c) Ranitidinhydrochlorid ist.

14. Verfahren zum Herstellen einer Arzneiform, **dadurch gekennzeichnet, dass** es folgende Schritte umfasst:
a) Befüllen einer ersten Kartusche mit der 3D-Druckfarbe nach einem der Ansprüche 1 bis 10, umfassend einen ersten Wirkstoff bei einer Temperatur zwischen 34 - 40 °C;
b) Einführen der Kartusche aus Schritt a) in den Extruder eines 3D-Druckers;
c) Einstellen der Temperatur des Extruders des Druckers auf 35 - 37 °C und der Temperatur eines Druckbetts auf 15 - 19 °C;
d) Drucken der Arzneiform.

15. Verfahren zum Herstellen einer Arzneiform nach Anspruch 14, **dadurch gekennzeichnet, dass** es zusätzlich folgende Schritte umfasst:
e) Befüllen einer zweiten Kartusche mit der 3D-Druckfarbe nach einem der Ansprüche 1 bis 10 mit einem zweiten Wirkstoff bei einer Temperatur zwischen 34 - 40 °C,
f) Einführen der Kartusche aus Schritt e) in den Extruder eines 3D-Druckers;
g) Einstellen der Temperatur des Extruders des Druckers auf 35 - 37 °C und der Temperatur eines Druckbetts auf 15 - 19° C;
h) Drucken der Arzneiform.

16. Arzneiform, erhalten nach Anspruch 14 oder 15, zur Anwendung bei der Behandlung einer Krankheit.

17. Arzneiform, erhalten nach Anspruch 14 oder 15, zur Anwendung bei der Behandlung von Zwölffingerdarmgeschwüren, gutartigen Magengeschwüren, dem Zollinger-Ellison-Syndrom, der Vorbeugung von wiederkehrenden Blutungen bei Patienten mit einem blutenden Geschwür, Ösophagitis peptica, der Vorbeugung von gastrointestinalen Blutungen, der Behandlung von Patienten mit dem Risiko einer Säureaspiration oder von Geburtshilfepatientinnen während der Entbindung bei Erwachsenen.

18. Arzneiform, erhalten nach Anspruch 14 oder 15, zur Anwendung bei der Behandlung von Ulcus pepticum und bei der Behandlung von gastroösophagealem Reflux in der Pädiatrie.

## Revendications

1. Composition comprenant un principe actif pharmaceutique, au moins trois hydrocolloïdes et de l'eau purifiée, **caractérisée en ce que** les trois hydrocolloïdes sont la gélatine, l'iota-carraghénane et la gomme xanthane, dans laquelle la composition est une encre d'impression 3D.

2. Composition selon la revendication 1, **caractérisée en ce que** la composition comprend en outre un excipient choisi parmi le groupe constitué par l'amidon de maïs, un édulcorant, une essence et un colorant alimentaire ou une combinaison de ceux-ci.

3. Composition selon l'une quelconque des revendications 1 ou 2, **caractérisée en ce que** le principe actif pharmaceutique est un principe actif soluble dans l'eau ou un principe actif en suspension.

4. Composition selon la revendication 3, **caractérisée en ce que** le principe actif soluble dans l'eau est choisi parmi le groupe constitué par le chlorhydrate de ranitidine, l'acétate de flécaïnide, le maléate d'énalapril, la n-acétylcystéine, le paracétamol, le dichlorhydrate de cétirizine, la digoxine, le sulfate de gentamicine et le sulfate de néomycine.

5. Composition selon l'une quelconque des revendications 1 à 4, **caractérisée en ce que** l'encre comprend 2 - 4% p/p d'iota-carraghénane par rapport au poids total de l'encre.

6. Composition selon l'une quelconque des revendications 1 à 5, **caractérisée en ce que** la composition comprend 0,25 - 0,50% p/p de gomme xanthane par rapport au poids total de la composition.

7. Composition selon l'une quelconque des revendications 1 à 6, **caractérisée en ce que** la composition comprend 5 - 8% p/p de gélatine par rapport au poids total de la composition.

8. Composition selon l'une quelconque des revendications 2 à 7, **caractérisée en ce que** la composition comprend entre 5 et 8% p/p d'amidon de maïs par rapport au poids total de la composition.

9. Composition selon l'une quelconque des revendications 1 à 8, **caractérisée en ce que** le principe actif pharmaceutique est le chlorhydrate de ranitidine.

10. Composition selon les revendications 1 à 9, **caractérisée en ce que** la composition comprend 3 - 15% de chlorhydrate de ranitidine, 2% - 4% p/p d'iota-carraghénane, 0,25 - 0,50% p/p de gomme xanthane, 5 - 8% p/p de gélatine de type A, 0 - 3,5% p/p d'un édulcorant liquide à l'exclusion de 0, 0,1 - 0,5% p/p d'essence de fraise, 0 - 0,5% à l'exclusion de 0 d'un colorant alimentaire, 5 - 8% p/p d'amidon de maïs et de l'eau purifiée, où les pourcentages sont par rapport au poids total de la composition.

11. Procédé pour la préparation d'une composition pharmaceutique sous forme d'encre d'impression 3D, **caractérisé en ce qu'**il comprend les étapes suivantes :
a) mise en contact de 5 - 8% p/p d'une gélatine et 5-7 fois d'eau purifiée par rapport au poids de la gélatine, à température ambiante pendant 10 - 20 minutes pour obtenir une gélatine hydratée ;
b) chauffage de la gélatine hydratée de l'étape a) à 40 ± 2°C ;
c) ajout d'une solution de principe actif pharmaceutique (API) dans l'eau purifiée à température ambiante à une solution d'i-carraghénane à 2 - 4% p/p et gomme xanthane à 0,25 - 0,50% p/p, les deux pourcentages étant par rapport au poids total de l'encre, dans l'eau purifiée à 35 - 40°C et agitation pour obtenir un mélange homogène ;
d) ajout de la gélatine hydraté de l'étape b) au mélange homogène de l'étape c) pour obtenir un deuxième mélange ;
e) ajout au deuxième mélange de l'étape d) de 0,1 - 0,5% p/p d'une essence, 0 - 3,5% p/p à l'exclusion de 0 d'un édulcorant, 0 - 0,5% p/p à l'exclusion de 0 d'un colorant alimentaire, où les pourcentages sont par rapport au poids total de l'encre, et mélange douce ;
f) incubation pendant 30 - 60 minutes à 40 - 45°C pour obtenir l'encre d'impression.

12. Procédé selon la revendication 11, **caractérisé en ce que** 5 - 8% p/p d'amidon de maïs par rapport au poids total de l'encre sont ajoutés dans l'étape c).

13. Procédé selon les revendications 11 et 12, **caractérisé en ce que** le principe actif dans l'étape c) est le chlorhydrate de ranitidine.

14. Procédé pour la fabrication d'une forme pharmaceutique **caractérisé en ce qu'**il comprend les étapes de :
a) remplissage d'une première cartouche avec l'encre d'impression 3D selon l'une quelconque des revendications 1 à 10 comprenant un premier principe actif à une température d'entre 34 - 40°C ;
b) introduction de la cartouche de l'étape a) dans l'extrudeuse d'une imprimante 3D ;
c) réglage de la température de l'extrudeuse de l'imprimante à 35 - 37°C et la température d'un lit d'impression à 15 - 19°C ;
d) impression de la forme pharmaceutique.

15. Procédé pour la fabrication d'une forme pharmaceutique selon la revendication 14, **caractérisé en ce qu'**il comprend les étapes additionnelles de :
e) remplissage d'une deuxième cartouche avec l'encre d'impression 3D selon l'une quelconque des revendications 1 à 10 avec un deuxième principe actif à une température d'entre 34 - 40°C,
f) introduction de la cartouche de l'étape e) dans l'extrudeuse d'une imprimante 3D ;
g) réglage de la température de l'extrudeuse de l'imprimante à 35 - 37°C et la température d'un lit d'impression à 15 -19° C ;
h) impression de ladite forme pharmaceutique.

16. Forme pharmaceutique obtenue dabs les revendications 14 ou 15, pour son utilisation dans le traitement d'une maladie.

17. Forme pharmaceutique obtenue dans les revendications 14 ou 15, pour son utilisation dans le traitement de l'ulcère duodénal, de l'ulcère gastrique bénin, du syndrome de Zollinger-Ellison, la prévention d'hémorragies récurrentes chez des patients avec une ulcère hémorragique, l'oesophagite peptique, la prévention d'hémorragies gastrointestinales, le traitement des patients à risque d'aspiration acide ou patientes obstétricales pendant l'accouchement chez les adultes.

18. Forme pharmaceutique obtenue dans les revendications 14 ou 15, pour son utilisation dans le traitement de l'ulcère peptique et dans le traitement du reflux gastro-oesophagien en pédiatrie.
